# EUROPÄISCHE PATENTSCHRIFT

(11) **EP 2 996 738 B1**
(45) Veröffentlichungstag und Bekanntmachung des Hinweises auf die Patenterteilung: **26.12.2018**
(21) Anmeldenummer: 14727736.2
(22) Anmeldetag: 14.05.2014
(51) Int. Cl.: A61M 1/16, G06F 19/00, G16H 40/20

(54) **VORRICHTUNG UND VERFAHREN ZUR OPTIMIERUNG DES ENERGIEVERBRAUCHS IN EINER MEDIZINISCHEN EINRICHTUNG**
DEVICE AND METHOD FOR OPTIMIZING THE ENERGY CONSUMPTION IN A MEDICAL APPARATUS
DISPOSITIF ET PROCÉDÉ D'OPTIMISATION DE LA CONSOMMATION D'ÉNERGIE D'UN ÉQUIPEMENT MÉDICAL

(30) Priorität: 17.05.2013 EP 13002607
(43) Veröffentlichungstag der Anmeldung: 23.03.2016
(73) Patentinhaber: Fresenius Medical Care Deutschland GmbH, 61352 Bad Homburg (DE)
(72) Erfinder: BRAUN, Christian, D - 67434 Neustadt a. d. Weinstraase (DE); GRÄFE, Marco, 61350 Bad Homburg (DE)
(74) Vertreter: Dreyhsig, Jörg
(86) Internationale Anmeldenummer: PCT/EP2014/059820
(87) Internationale Veröffentlichungsnummer: WO 2014/184227

(56) Entgegenhaltungen:
- EP-A1- 0 436 855
- US-A- 3 809 241
- US-A- 5 624 572
- US-A1- 2009 206 023
- US-A1- 2012 308 431
- US-B2- 7 976 711

## Beschreibung

### Technisches Gebiet

Die Erfindung betrifft das Gebiet der Optimierung des Energieverbrauchs in einer medizinischen Einrichtung, insbesondere die Optimierung des Energieverbrauchs in einer Dialyseklinik.

Der Betrieb medizinischer Einrichtungen erfordert die Bereitstellung von Energieressourcen wie Wärme und elektrischer Energie, einerseits für den Betrieb der medizinischen Geräte selber, andererseits zur Vorbereitung des Betriebs der medizinischen Geräte sowie für die Bereitstellung von Stoffen, die bei dem Betrieb der medizinischen Geräte benötigt werden, sowie zur Vorbereitung der hierzu verwendeten Geräte. So ist der Betrieb von Dialysegeräten in einer Dialyseklinik mit einem Verbrauch elektrischer Energie für den Betrieb der Dialysegeräte verbunden, aber auch mit dem Verbrauch von Wärme und elektrischer Energie zur Vorbereitung der Dialysegeräte, etwa zur Desinfektion. Die Bereitstellung von Dialysierflüssigkeit ist mit dem Verbrauch von elektrischer Energie und Wärme für die Bereitstellung von Dialysierflüssigkeit, einschließlich der Ausgangsstoffe für die Dialysierflüssigkeit wie Reinstwasser, das auch als Permeat oder RO- Wasser bezeichnet wird und durch Umkehrosmose (reverse osmosis) gewonnen wird. Die Vorbereitung der RO Anlagen für den Betrieb umfasst eine Desinfektion mit Heisswasser oder einem anderen Heissdesinfektionsmittel, verbunden mit einem entsprechenden Verbrauch von Wärme oder elektrischer Energie. US 2012/0308431 offenbart ein bekanntes System zur Versorgung mehrerer Dialysegeräte mit Reinstwasser, wobei eine Desinfektion mit Heisswasser vorgesehen ist.

Medizinische Einrichtungen müssen Anforderungen hinsichtlich des Erreichens von Umweltzielen und der Aufwendungen für ihren Betrieb genügen.

Es ist daher die Aufgabe der vorliegenden Erfindung ein Verfahren zur Optimierung des Energieverbrauchs in einer medizinischen Einrichtung sowie ein entsprechendes System zur Verfügung zu stellen.

### Zusammenfassung

Diese Aufgabe wird gelöst durch ein Verfahren zur Optimierung eines Energieverbrauchs einer medizinischen Einrichtung nach Anspruch 1 sowie durch ein System zur Optimierung eines Energieverbrauchs in einer medizinischen Einrichtung nach Anspruch 5.

Vorteilhafte Ausgestaltungen sind in den abhängigen Ansprüchen angegeben.

Im Einklang mit der Lehre der vorliegenden Erfindung wird ein System zur Optimierung des Energieverbrauchs einer medizinischen Einrichtung mit einem Medizingerät sowie einen weiteren Energieverbraucher bereitgestellt. Das System umfasst Folgendes:
ein klinisches Informationssystem zum Bereitstellen von Behandlungsplanungsinformation, die einen zeitlichen Ablauf für die Nutzung des Medizingeräts vorgibt,
eine Datenbank zur Bereitstellung von Wechselwirkungsinformation, die eine Wechselwirkung zwischen der Nutzung des Medizingeräts und dem weiteren Energieverbraucher vorgibt, sowie eine mit dem klinischen Informationssystem und mit der Datenbank verbundene Verarbeitungseinheit, die angepasst ist Steuerinformation zum Ansteuern des weiteren Energieverbrauchers anhand der Behandlungsplanungsinformation und der Wechselwirkungsinformation bereitzustellen.

In einer Ausführungsform ist das medizinische Gerät eine Dialysemaschine, die im Betrieb eine Wärmequelle darstelle und der weitere Energieverbraucher ist eine Heizung. Die Wechselwirkungsinformation umfasst in dieser Ausführungsform eine Angabe der mit dem Betrieb der Dialysemaschine verbundenen Wärmeproduktion. Die Steuerinformation für die Heizung wird in dieser Ausführungsform unter Berücksichtigung von Behandlungsplanungsinformation für die Dialysemaschine, sowie unter Berücksichtigung der Angabe zur Wärmeproduktion der Dialysemaschine bereitgestellt. So kann etwa die Heizung zu einem bestimmten Zeitpunkt, der sich aus der Behandlungsplanungsinformation ergibt um einen Betrag abgesenkt werden, der der Angabe der mit dem Betrieb der Dialysemaschine verbundenen Wärmeproduktion entspricht.

In einer weiteren Ausführungsform ist das medizinische Gerät eine Dialysemaschine und der weitere Energieverbraucher ist eine Heißdesinfektionsanlage zur Heißdesinfektion einer Anlage zum Bereitstellen von RO Wasser zur Bereitstellung von Dialysierflüssigkeit. Die Behandlungsplanungsinformation gibt in diesem Fall eine Zeitspanne an, um die die Heißdesinfektion der Dialysebehandlung vorausgehen muss. Die Steuerinformation für die Heißdesinfektionsanlage wird in dieser Ausführungsform unter Berücksichtigung von Behandlungsplanungsinformation für die Dialysemaschine, sowie unter Berücksichtigung der Angabe der Zeitspanne, um die die Heißdesinfektion der Behandlung vorausgeht bereitgestellt. So kann etwa die Heißdesinfektionsanlage zu einem bestimmten Zeitpunkt, der sich aus der Behandlungsplanungsinformation sowie der angegebenen Zeitspanne ergibt in Betrieb genommen werden.

### Kurzbeschreibung der Zeichnungen

Figur 1 zeigt ein System zur Optimierung des Energieverbrauchs in einer medizinischen Einrichtung
Figur 2 zeigt ein weiteres System zur Optimierung des Energieverbrauchs in einer medizinischen Einrichtung

### Detaillierte Beschreibung der Zeichnungen

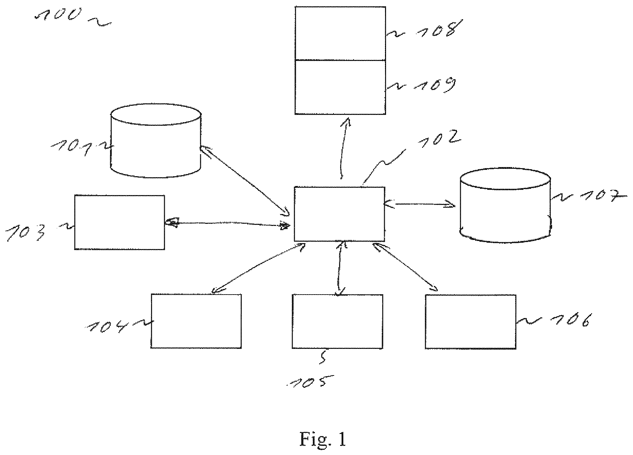

Figur 1 zeigt ein System 100 zur Optimierung des Ressourcenverbrauchs und/ oder des Energieverbrauchs in einer medizinischen Einrichtung, vorzugsweise in einer Dialyseklinik in der medizinische Geräte einschließlich Dialysemaschinen betrieben werden. In der medizinischen Einrichtung sind außerdem weitere Energieverbraucher vorhanden, wie Heizungen, Desinfektionsanlagen für Umkehrosmoseanlagen, und Umkehrosmoseanlagen, die durch Umkehrosmose Reinstwasser als Ausgangssubstanz für Dialysierflüssigkeit bereitstellen.

Das System 100 umfasst eine Verarbeitungseinheit 102, zur Bereitstellung von Steuerinformation für die Medizingeräte sowie für die weiteren Energieverbraucher innerhalb der Dialyseklinik, insbesondere der Heizungen, und der Desinfektionsanlagen.

In dem Register 103 sind Merkmale der medizinischen Geräte einschließlich der Dialysemaschinen abgelegt, die mit dem Energie- oder Ressourcenmanagement in Verbindung stehen, wie der Wärmeverbrauch, der Verbrauch an elektrischer Energie, die Wärmeabgabe oder der Wasserverbrauch.

In dem Register 104 sind auf die Haustechnik bezogene Parameter abgelegt einschließlich einer Jahresheizkurve oder eines Jahresenergiebedarfs,
In dem Register 105 sind Parameter, die verschiedene Ressourcenversorger der Dialyseklinik betreffen, abgelegt, etwa der die Jahresheizkurve des Wärmeversorgers oder die Tagesverlaufskurve eines Energieverbrauchs eines Energieversorgers einschließlich von tageszeitabhängigen Energieverbrauchspreisen.

In dem Register 106 sind auf die medizinische Einrichtung bezogene Verlaufskurven von Verbräuchen innerhalb der medizinischen Einrichtung abgelegt, etwa über einen bestimmten Zeitverlauf in der medizinischen Einrichtung gemessene Verbrauchkurven für den Verbrauch an elektrischer Energie, von Wasser und/ oder von Heizenergie.

Die in den Registern 103, 104, 105, sowie 106 verwaltete Information betrifft eine Wechselwirkung zwischen der Nutzung der medizinischen Geräte einschließlich der Dialysemaschinen und weiteren Energieverbrauchern innerhalb der Dialyseklinik.

In dem klinischen Informationssystem 107 wird Planungsinformation bereitgestellt, einschließlich Belegungsplänen für die Belegung von Dialysemaschinen mit Patienten, Behandlungsplanungsinformation sowie von Schichtpläne betreffender Information. Behandlungsplanungsinformation umfasst Zeitangaben, zu welchem Zeitpunkt die Behandlung eines bestimmten Patienten mit einer Bestimmten Maschine vorgesehen ist.

Die Datenbank 101 dient der Verwaltung der medizinischen Geräte einschließlich der Dialysemaschinen.

Die Verarbeitungseinheit 102 kann auf die Behandlungsplanungsinformation des klinischen Informationssystems 107 sowie auf die Register 103, 104, 105 und 106 zugreifen.

Anhand der Behandlungsplanungsinformation des klinischen Informationssystems sowie anhand von Wechselwirkungsinformation die eine Wechselwirkung zwischen der Nutzung des Medizingeräts und dem weiteren Energieverbraucher vorgibt stellt die Verarbeitungseinheit 102 Steuerinformation oder Einstellungsempfehlungen 109 für Medizingeräte einschließlich Dialysemaschinen sowie oder Steuerinformation oder Einstellungsempfehlungen 108 für weitere Verbraucher zur Optimierung des Ressourcenverbrauchs einschließlich des Wasserverbrauchs sowie des Verbrauchs von Wärmeenergie oder elektrischer Energie zusammen.

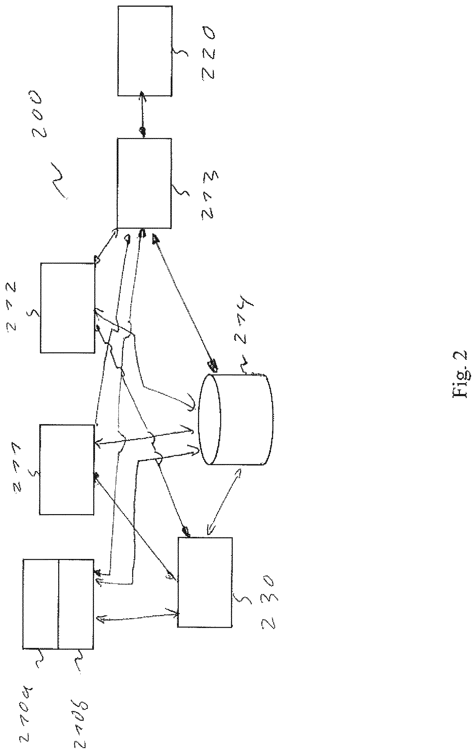

Figur 2 zeigt ein weiteres System 200 zur Optimierung des Energieverbrauchs in einer medizinischen Einrichtung.

Das System 200 weist medizinische Geräte 211 einschließlich Dialysemaschinen auf, deren Betrieb mit dem Verbrauch von Ressourcen und von Energie einschließlich elektrischer Energie und/ oder Wärmeenergie verbunden ist.

Die Dialyseklinik weist weiterhin eine Haustechnik 212 auf, die Ressourcen für den Betrieb der medizinischen Einrichtung bereitstellt und die mit weiteren Verbrauchern von Ressourcen einschließlich Wasser, elektrischer Energie und Wärmeenergie verbunden ist.

Die weiteren Verbraucher sind mit Aktoren 210a und Sensoren 210b ausgerüstet, wobei die Sensoren Istzustände des Ressourcenverbrauchs der Verbraucher erfassen, einschließlich eines momentanen Wasserverbrauchs, eines momentanen Verbrauchs an elektrischer Energie und/ oder eines momentanen Verbrauchs an Wärmeenergie. Die Sensoren 210b steuern die Verbraucher und geben dabei Sollwerte für den Ressourcenverbrauch vor, einschließlich eines Sollwerts für den Wasserverbrauch, eines Sollwerts für den Verbrauch von elektrischer Energie und/ oder eine Sollwerts für den Verbrauch von Wärmeenergie.

Eine sogenannte Middleware 213 ist mit den Aktoren 210a, den Sensoren 210b, mit den medizinischen Geräten 211 mit der Haustechnik 212 sowie mit einer Leitstelle 220 verbunden. Die Middleware dient der Anpassung zwischen Datenformaten der Leitstelle 220 einerseits und den Datenformaten der Aktoren 210a, der Sensoren 210b, der medizinischen Geräten 212 und der Haustechnik 212 andererseits. Die Verbindung der Middleware 213 mit den Aktoren 210a, den Sensoren 210b, mit den medizinischen Geräten 211 mit der Haustechnik 212 kann über ein LAN (Local Area Network) oder ein W-LAN (Wireless Local Area Network) erfolgen. Wechselwirkungsinformation, die eine Wechselwirkung zwischen dem Ressourcenverbrauch eines bestimmten Medizingeräts sowie eines bestimmten weiteren Verbraucher vorgibt, ist in der Datenbank 214 abgelegt.

Die Datenbank 214 ist über Datenverbindungen mit den medizinischen Geräten 212, mit der Haustechnik 212, sowie mit den Aktoren 210a und den Sensoren 210b verbunden. Die Datenbank kann außerhalb der medizinischen Einrichtung betrieben werden. In diesem Fall können diese Datenverbindungen eine Internetverbindung, eine Verbindung über ein Mobilfunknetz oder eine M2M (machine to machine) Verbindung umfassen.

Die Leitstelle 220 kann eine Leitstelle innerhalb der medizinischen Einrichtung sein, oder einer alternativen Ausführungsform kann eine zentrale Leitstelle 230 vorgesehen sein, die entsprechende Funktionen ausführt, wobei die Leitstelle vorzugsweise als landesweite Leitstelle oder als Leitstelle für medizinische Einrichtungen in verschiedenen Ländern dient.

## Patentansprüche

1. Verfahren zur Optimierung des Energieverbrauchs einer medizinischen Einrichtung umfassend ein Medizingerät sowie einen weiteren Energieverbraucher wobei das Verfahren die folgenden Schritte umfasst:
Bereitstellen von Behandlungsplanungsinformation, die einen zeitlichen Ablauf für die Nutzung des Medizingeräts vorgibt,
Bereitstellung von Wechselwirkungsinformation, die eine Wechselwirkung zwischen der Nutzung des Medizingeräts und dem weiteren Energieverbraucher vorgibt,
Bereitstellen von Steuerinformation zum Ansteuern des weiteren Energieverbrauchers anhand der Behandlungsplanungsinformation und der Wechselwirkungsinformation, wobei das medizinische Gerät im Betrieb eine Wärmequelle darstellt, wobei der weitere Energieverbraucher eine Heizung ist,
wobei die Wechselwirkungsinformation eine mit dem Betrieb des medizinischen Geräts verbundene Wärmeproduktion umfasst und wobei die Wechselwirkungsinformation weiterhin einen zeitlichen Bezug zwischen dem Betrieb der medizinischen Vorrichtung und dem Betrieb des weiteren Energieverbrauchers vorgibt, und wobei der Schritt des Bereitstellens von Wechselwirkungsinformation weiterhin folgendes umfasst:
Ablegen von Parametern, einschließlich einer Jahresheizkurve eines Wärmeversorgers oder einer Tagesverlaufskurve eines Energieverbrauchs eines Energieversorgers in einem einen Ressourcenversorger der Dialyseklinik betreffenden Register, oder Ablegen von Verbräuchen innerhalb der medizinischen Einrichtung, einschließlich eines bestimmten Zeitverlaufs einer in der medizinischen Einrichtung gemessenen Verbrauchkurve für den Verbrauch an elektrischer Energie, von Wasser oder von Heizenergie in einem Register für auf die medizinische Einrichtung bezogenen Verlaufskurven, und wobei der weitere Energieverbraucher unmittelbar unter Verwendung der Steuerinformation angesteuert wird.

2. Verfahren nach Anspruch 1, wobei der weitere Energieverbraucher ein Hilfsgerät für den Betrieb des medizinischen Geräts darstellt, und wobei die Wechselwirkungsinformation eine zeitliche Vorgabe für das Hilfsgerät in Bezug auf den Behandlungsablauf umfasst.

3. Verfahren nach einem der vorangehenden Ansprüche, wobei das medizinische Gerät ein Dialysegerät ist.

4. Verfahren nach einem der vorangehenden Ansprüche, wobei die Steuerinformation auf einer Anzeigevorrichtung, insbesondere als Einschaltempfehlung auf einem Display, dargestellt wird.

5. System zur Optimierung des Energieverbrauchs einer medizinischen Einrichtung mit einem Medizingerät sowie einen weiteren Energieverbraucher, wobei das medizinische Gerät im Betrieb eine Wärmequelle darstellt, und der weitere Energieverbraucher eine Heizung ist, und wobei das System folgendes umfasst:
ein klinisches Informationssystem zum Bereitstellen von Behandlungsplanungsinformation, die einen zeitlichen Ablauf für die Nutzung des Medizingeräts vorgibt,
eine Datenbank zur Bereitstellung von Wechselwirkungsinformation, die eine Wechselwirkung zwischen der Nutzung des Medizingeräts und dem weiteren Energieverbraucher vorgibt,
einen einen Ressourcenversorger der Dialyseklinik betreffenden Register, in dem eine Jahresheizkurve eines Wärmeversorgers oder eine Tagesverlaufskurve eines Energieverbrauchs eines Energieversorgers als Wechselwirkungsinformation abgelegt ist, und/ oder einen Register für auf die medizinische Einrichtung bezogene Verlaufskurven, in dem Parameter zu Verbräuchen innerhalb der medizinischen Einrichtung, einschließlich eines bestimmten Zeitverlaufs einer in der medizinischen Einrichtung gemessenen Verbrauchkurve für den Verbrauch an elektrischer Energie, von Wasser oder von Heizenergie als Wechselwirkungsinformation abgelegt ist,
sowie eine mit dem klinischen Informationssystem und mit der Datenbank verbundene Verarbeitungseinheit, die angepasst ist, Steuerinformation zum Ansteuern des weiteren Energieverbrauchers anhand der Behandlungsplanungsinformation und der Wechselwirkungsinformation bereitzustellen, so dass der weitere Energieverbraucher unmittelbar unter Verwendung der Steuerinformation angesteuert wird, und wobei die Wechselwirkungsinformation weiterhin folgendes umfaßt:
eine mit dem Betrieb des medizinischen Geräts verbundene Wärmeproduktion sowie einen zeitlichen Bezug zwischen dem Betrieb der medizinischen Vorrichtung und dem Betrieb des weiteren Energieverbrauchers, insbesondere der Heizung.

6. System nach Anspruch 5, angepasst zur Durchführung eines Verfahrens nach einem der Ansprüche 2 - 4.

## Claims

1. A method for optimizing energy consumption by a medical apparatus comprising a medical apparatus and another energy consumer, wherein the method comprises the following steps:
supplying treatment planning information, which stipulates a chronological sequence for use of the medical apparatus,
supplying interaction information, which stipulates an interaction between the use of the medical apparatus and the additional energy consumers,
supplying control information for controlling the additional energy consumer on the basis of the treatment planning information and the interaction information, wherein the medical apparatus is a heat source during operation, wherein the additional energy consumer is a heater, wherein the interaction information comprises heat production associated with operation of the medical apparatus, and wherein the interaction information additionally comprises a chronological relationship between operation of the medical apparatus and operation of the additional energy consumer, and wherein the step of supplying interaction information additionally comprises the following:
storing parameters, including an annual heating curve of a heat provider or a daily heating curve of the energy consumption by an energy provider in a register pertaining to a resource provider of the dialysis clinic, or storing consumption data within the medical apparatus, including a certain interval of time of a consumption curve measured in the medical apparatus for consumption of electrical energy, water or heating energy in a register for curves based on the medical apparatus, and wherein the additional energy consumers are controlled directly by using the control information.

2. The method according to claim 1, wherein the additional energy consumer is an auxiliary device for operation of the medical apparatus, and wherein the interaction information comprises a time specification for the auxiliary device with respect to the course of treatment.

3. The method according to any one of the preceding claims, wherein the medical apparatus is a dialysis machine.

4. The method according to any one of the preceding claims, wherein the control information displayed on a display device, in particular as a recommended activation on a display screen.

5. A system for optimizing the energy consumption of a medical apparatus having a medical apparatus as well as an additional energy consumer, wherein the medical apparatus is a heat source during operation, and the additional energy consumer is a heater, and wherein the system comprises the following:
a clinical information system for supplying treatment planning information, which stipulates a chronological course for use of the medical apparatus,
a database for supplying interaction information, which stipulates an interaction between use of the medical apparatus and the additional energy consumer, a register pertaining to a resource provider of the dialysis clinic, in which an annual heating curve of a heat provider or a daily curve of the energy consumption of an energy provider is stored as interaction information and/or a register for curves based on the medical apparatus, in which parameters about consumption within the medical apparatus, including a certain course of time of a consumption curve, measured in the medical apparatus over time, for consumption of electric energy, water or heating energy is stored as interaction information,
as well as a processing unit associated with the clinical information system and with the database, adapted to supply control information for controlling the additional energy consumer on the basis of the treatment planning information and the interaction information, so that the additional energy consumer is controlled directly by using the control information, wherein the interaction information additionally comprises the following:
heat production associated with operation of the medical apparatus as well as a time reference between operation of the medical apparatus and operation of the additional energy consumer, in particular the heating.

6. The system according to claim 5, adapted for carrying out the method according to any one of claims 2 to 4.

## Revendications

1. Procédé d'optimisation de la consommation d'énergie d'un équipement médical comprenant un appareil médical et un autre consommateur d'énergie, ce procédé comprenant les étapes suivantes :
mise à disposition d'informations de programme de traitement préconisant un déroulement temporel pour l'utilisation de l'appareil médical,
mise à disposition d'informations d'interaction préconisant une interaction entre l'utilisation de l'appareil médical et l'autre consommateur d'énergie,
mise à disposition d'informations de commande pour la commande de l'autre consommateur à partir des informations de programms de traitement et des informations d'interaction, l'appareil médical constituant en cours d'utilisation une source de chaleur, l'autre consommateur d'énergie étant un chauffage, les informations d'interaction comprenant une production de chaleur liée au fonctionnement de l'appareil médical et les informations d'interaction préconisant en outre une relation temporelle entre le fonctionnement du dispositif médical et le fonctionnement de l'autre consommateur d'énergie, et l'étape de mise à disposition d'informations d'interaction comprenant en outre ce qui suit :
enregistrement de paramètres, y compris une courbe de chauffage annuelle d'un fournisseur de chaleur ou une courbe d'évolution quotidienne d'une consommation de chaleur d'un fournisseur d'énergie dans un registre concernant un fournisseur de ressources de la clinique de dialyse, ou enregistrement de consommateurs dans l'équipement médical, y compris une certaine évolution temporelle d'une courbe de consommation mesurée dans l'équipement médical pour la consommation d'énergie électrique, d'eau ou d'énergie de chauffage dans un registre pour les courbes d'évolution relatives à l'équipement médical, et l'autre consommateur d'énergie étant commandé directement en utilisant les informations de commande.

2. Procédé selon la revendication 1, l'autre consommateur d'énergie constituant un appareil auxiliaire pour le fonctionnement de l'appareil médical, et des informations d'interaction comprenant une préconisation temporelle pour l'appareil auxiliaire en relation avec le déroulement du traitement.

3. Procédé selon une des revendications précédentes, dans lequel l'appareil médical est un appareil de dialyse.

4. Procédé selon une des revendications précédentes, dans lequel les informations de commande sont visualisées sur un dispositif d'affichage, en particulier sous forme de recommandations d'activation sur un écran.

5. Système d'optimisation de la consommation d'énergie d'un équipement médical comprenant un appareil médical et un autre consommateur d'énergie, l'appareil médical constituant en cours de fonctionnement une source de chaleur, et l'autre consommateur d'énergie étant un chauffage, et le système comprenant ce qui suit :
un système d'information clinique pour la mise à disposition d'informations de programme de traitement préconisant un déroulement temporel pour l'utilisation de l'appareil médical,
une banque de données pour la mise à disposition d'informations d'interaction préconisant une interaction entre l'utilisation de l'appareil médical et l'autre consommateur d'énergie,
un registre concernant un fournisseur de ressources de la clinique de dialyse, dans lequel une courbe de chauffage annuelle d'un fournisseur de chaleur ou une courbe d'évolution quotidienne de consommation d'énergie d'un fournisseur d'énergie est enregistrée sous forme d'informations d'interaction, et/ou un registre pour les courbes d'évolution relatives à l'équipement médical, dans lequel des paramètres concernant des consommateurs sont enregistrés dans le dispositif médical, y compris une certaine évolution temporelle d'une courbe de consommation mesurée dans l'équipement médical pour la consommation d'énergie électrique, d'eau ou d'énergie de chauffage sous forme d'informations d'interaction,
ainsi qu'une unité de traitement raccordée au système d'information clinique et à la banque de données et qui est apte à mettre à disposition des informations de commande pour la commande de l'autre consommateur d'énergie à partir des informations de programme de traitement et des informations d'interaction, de sorte que l'autre consommateur d'énergie est commandé directement en utilisant les informations de commande, et les informations d'interaction comprenant en outre ce qui suit :
une production de chaleur liée au fonctionnement de l'appareil médical et une relation temporelle entre le fonctionnement du dispositif médical et le fonctionnement de l'autre consommateur d'énergie, en particulier du chauffage.

6. Système selon la revendication 5, adapté pour la réalisation d'un procédé selon une des revendications 2 à 4.
